# EUROPEAN PATENT APPLICATION

(11) **EP 2 568 047 A1**
(43) Date of publication of application: **13.03.2013**
(21) Application number: 11306110.5
(22) Date of filing: 07.09.2011
(51) Int. Cl.: C12N 15/82, A01H 5/00

(54) **New parthenocarpic plants with modified expression of auxin response factors and the microRNAs inducing said modified expression**

(71) Applicant: Institute National Polytechnique de Toulouse, 31000 Toulouse (FR)
(72) Inventor: Bouzayen, Mondher, 31320 AUZEVILLE-TOLOSANE (FR); Zouine, Mohamed, 31290 VILLEFRANCHE DE LAURAGAIS (FR); Pech, Jean-claude, 31400 TOULOUSE (FR); Latche, Alain, 31100 TOULOUSE (FR)
(74) Representative: Regimbeau

(57) **Abstract**

The present invention is related to a flowering plant expressing a DNA sequence coding for a microRNA, wherein the microRNA is specific to members of the Auxin-Reponse Factor (ARF) gene family and decreases the expression level of said ARFs when expressed. In particular said microRNA is specific of a functional equivalent of ARF2, ARF3, ARF4, ARF7, ARF10 or ARF17 from *Solanum lycopersicum.* The invention is also related to the specific microRNA, and to a method for producing a parthenocarpic flowering plant.

## Description

### INTRODUCTION

Fruit development is a genetically programmed process unique to flowering plants and the transition from flower to fruit, the so-called fruit set, is an essential step towards fruit initiation. Fruit organogenesis originates from a flower primordial and in most cases fleshy fruit is the result of the development of the ovary. The mature flower can either be fertilized to develop into a fruit or, in the absence of successful pollination, the flower enter the abscission process. Therefore, the onset of the development of an ovary into fruit constitutes an essential mechanism for fruit production. However, the signals that trigger fruit growth after fertilization remain poorly understood. Unfavorable conditions, such as extreme temperatures, may prevent pollination and hence fruit set. Moreover, mild temperature stresses are known to lead to loss of pollen viability which results in the production of under-fertilized puffy fruits of poor quality, while severe temperature stress can completely abolish fruit set.

In tomato (*Solanum lycopersicum*) and many other species, a major limiting factor for fruit set is the extreme sensitivity of microsporogenesis and pollination to moderate fluctuations in temperature and humidity. In this context, parthenocarpy, the growth of the ovary into a seedless fruit in the absence of pollination and fertilization, becomes an important trait that provides a mean to overcome the problems of low fruit set in harsh environmental conditions.

Fertilization-independent fruit set can occur either naturally or by induction via exogenous application to the flower of phytohormones, such as auxin and gibberellins (GAs). In support to the essential role of plant hormones, elevated endogenous levels of auxin and GA have been observed during parthenocarpic fruit set and accordingly, parthenocarpy can be induced in diverse agricultural species not only by the exogenous application of auxins, cytokinins, or GAs but also by altering their biosynthesis and response pathways.

However, the exact mechanisms by which auxin promote fruit initiation and the nature of the genes that control fruit set remain an open question, and the molecular actors controlling the fruit initiation process are far from being all identified.

Auxin signalling and response are mediated by transcriptional regulators from the ARF (Auxin Response Factors) type and hence, these transcription factors become potential candidates in relaying the hormone action involved in fruit set.

### PRIOR ART

Twenty-one ARF genes were identified and cloned from tomato plants, and their ability to control the expression of auxin-responsive genes at the transcriptional level investigated in a dedicated single cell system. Structural analysis the isolated genes performed *in silico* indicated that all the Sl-ARF encoded proteins contain a typical DBD domain and the two conserved protein/protein interaction domains required for the binding to the Aux/IAA proteins. Throughout the process of natural fruit set, both ARFs and Aux/IAAs display a dramatic shift in their expression, suggesting an active role of these transcriptional regulators in this developmental process. Notably, ARF4, ARF8 and ARF18 are upregulated, although ARF3, ARF7, ARF10 and ARF17 are downregulated at the transitions anthesis/bud and post-anthesis/anthesis (Wang et al., 2009).

The genes encoding the transcriptional factors ARF, ie. mediating the response to auxin, have been found to be involved in parthenocarpy:
**●** Expression of an aberrant form of the *Arabidopsis* ARF8 gene in tomato has been found to cause parthenocarpy (Gorguet et al., 2008, *Theor Appl Genet*);
● Downregulation of mRNA levels of ARF7 in *Solanum lycopersicum,* by using interfering RNA, has been shown to form seedless fruits (de Jong et al., 2009 and de Jong et al., 2011).

ARF genes appear like good candidates for controlling the induction of parthenocarpy in flowering plants. However, the exact mechanisms of their regulation *in vivo* are not known yet.

### DESCRIPTION OF THE INVENTION

The present invention is related to a flowering plant, having a altered expression of an Auxin Response Factor (ARF), wherein said altered expression confers at least one of the following properties to the plant: fruit set independent from pollination/fertilization, fruit parthenocarpy, increased number of fruit, increased number of locules per fruit, modified fruit shape, increased seed size, enhanced sensory and nutritional traits.

Inventors have shown that the expression of some specific ARF genes is regulated at the post-transcriptional level by a set of small interfering RNAs called 'microRNA' or 'short RNA'. Short RNAs have been recently identified in tomato (see for review Dalmay, 2010, J. *Integrative Plant Biology*)*,* but this is the first report of microRNA targeting and modulating the expression of ARF genes.

Hence, the identified microRNA genes provide a new target for manipulating the fruit set process via genetic and biotechnological approaches, and more generally to manipulate the expression of ARF members.

The present invention relates to new flowering plants expressing a DNA sequence encoding a microRNA specific to a ARF gene, ie. regulating specifically the products of the ARF genes at the post-transcriptional level.

### DETAILLED DESCRIPTION OF THE INVENTION

The present invention is related to a parthenocarpic flowering plant expressing a DNA sequence coding for a microRNA, wherein the microRNA is specific to members of the Auxin-Response Factor (ARF) gene family and decreases the expression level of said ARFs when expressed.

### Definitions

Unless otherwise defined herein or below in the remainder of the specification, all technical and scientific terms used herein have meanings commonly understood by those of ordinary skill in the art to which the present invention belongs.

Before describing the present invention in detail, it is to be understood that this invention is not limited to particular assays or biological systems, which can, of course, vary. It is also to be understood that the terminology used herein is for the purpose of describing particular embodiments only, and is not intended to be limiting. As used in this specification and the appended claims, the singular forms "a", "an" and "the" include plural referents unless the content clearly dictates otherwise. Thus, for example, reference to "a component" can include a combination of two or more components; reference to "a cell" can include mixtures of two or more cells, and the like.

Although many methods and materials similar, modified, or equivalent to those described herein can be used in the practice of the present invention without undue experimentation, the preferred materials and methods are described herein. In describing and claiming the present invention, the following terminology will be used in accordance with the definitions set out below.

As used herein, the following terms may be used for interpretation of the claims and specification.

In the claims which follow and in the preceding description of the invention, except where the context requires otherwise due to express language or necessary implication, the word "comprise" or variations such as "comprises" or "comprising" is used in an inclusive sense, i.e. to specify the presence of the stated features but not to preclude the presence or addition of further features in various embodiments of the invention.

A flowering plant, also known as Angiospermae or Magnoliophyta, is a plant forming flowers and seeds. This Order is subdivided into 12 subclasses, 35 superorders, 87 orders, 40 suborders, 472 families, and 400 subfamilies. It includes in particular the Solanaceae and Cucurbitaceae families.

Solanaceae is a family of flowering plants that contains a number of important agricultural crops as well as many toxic plants. The family includes Datura (Jimson weed), Mandragora (mandrake), belladonna (deadly nightshade), Lycium barbarum (Wolfberry), Physalis philadelphica (Tomatillo), Physalis peruviana (Cape gooseberry flower), Capsicum (paprika, chili pepper), Solanum (potato, tomato, aubergine or eggplant), Nicotiana (tobacco), and Petunia (petunia). With the exception of tobacco (Nicotianoideae) and petunia (Petunioideae) most of the economically important genera are contained in the sub-family Solanoideae. The Solanaceae family is extensively utilized by humans. It is an important source of food, spice and medicine.

Cucurbitaceae is a plant family, also known as gourd family, which includes crops like cucumbers, squashes (including pumpkins), luffas, watermelons and melons.

The term 'parthenocarpic' characterizes a fruit without fertilization of ovules. The fruit is therefore seedless.

The term "expression" or "expressed" refers to the biosynthesis, from a DNA sequence, of a RNA and eventually of a protein, in the case of the RNA is translated. In particular, it means that the DNA sequence is under the control of a promoter functional in the cell plant expressing said DNA sequence.

A 'DNA sequence' or 'DNA fragment' designates a succession of nucleotides.

The terms "encoding" or "coding" refer to the process by which a polynucleotide, through the mechanisms of transcription and translation, produces a RNA sequence and eventually an amino-acid sequence. In the present invention, "a DNA sequence coding for a microRNA" specifically designates the mechanism of transcription, leading to a short molecule of RNA that will not be translated.

The term "promoter" refers to a DNA sequence necessary for the initiation of the transcription of a structural gene, i.e. comprising a site for RNA polymerase binding, and including the TATA box. Typically, a promoter is located in the 5'region of the DNA sequence, proximal to the transcriptional start site of the DNA fragment.

A 'DNA sequence' or 'DNA fragment' designates a succession of nucleotides.

The terms "encoding" or "coding" refer to the process by which a polynucleotide, through the mechanisms of transcription and translation, produces a RNA sequence and eventually an amino-acid sequence. In the present invention, "a DNA sequence coding for a microRNA" specifically designates the mechanism of transcription, leading to a short molecule of RNA that will not be translated.

The term « vector » refers to an extra-chromosomal element carrying genes or cassettes, that is usually in the form of a circular double-stranded DNA molecule.

A « transformation vector » comprises a DNA sequence that is expressed in a host cell. Typically, the DNA sequence is placed under the control of certain regulatory elements including promoters, tissue specific regulatory elements, and enhancers. Such DNA sequence is said to be "operably linked to" the regulatory elements.

The term « transformation » refers to the incorporation of exogenous nucleic acid by a plant cell. Genetic transformation of plants are now well known in the art, comprising introducing a new DNA fragment in a plant cell and then regenerating a plant form the cell. The new DNA fragment is preferably introduced with known techniques of particle bombardment and/or infection with a transformed Agrobacterium. Regeneration procedure are also well known in the art and documented for numerous plant today. The new DNA fragment is preferably integrated into the plant cell genome. New techniques of homologous recombination and gene replacement are also known today to be effective in plant cells.

A "transformed plant cell" is a cell that contains exogenous DNA, in particular a vector. This term also includes those cells that have been genetically engineered to contain exogenous DNA sequences in their chromosome.

A "transgenic plant" is a plant having one or more plant cells that contain exogenous DNA sequences. Plant tissues include differentiated and undifferentiated tissues or plants, including but not limited to roots, stems, shoots, leaves, pollen, seeds, tumor tissue, and various forms of cells and culture such as single cells, protoplasm, embryos, and callus tissue. The plant tissue may be in plant or in organ, tissue, or cell culture.

The term of "Auxin Response Factor" or "ARF" designates a transcription factor having highly conserved domains among all the factors of the family including a DNA binding domain, an activation/repression domain and protein/protein interaction domains. The Auxin Response Factor (ARF) are transcription factor proteins important for transducing the perception of auxin through to changes in gene expression (Guilfoyle et al. (1998) Plant Physiol. 1 18:341 -347). ARFs bind to auxin response cis-acting elements through an N-terminal DNA binding domain, while the carboxy-terminus contains two protein-protein interaction domains. In vitro homodimerization and heterodimerization suggest that combinatorial action of these proteins confers cell or tissue specificity in auxin responses (Kim et al. ( 1997) Proc. Natl. Acad. Sci. USA 94: 1 1786-1 1791 ; Ulmasov et al. (1997a) Science 276: 1865-1868; Ulmasov, et al. ( 1997b) Plant Cell 9: 1963- 1971 ; Ulmasov et al. ( 1999) Plant J. 19:309-319).

MicroRNAs (miRNAs) are a class of regulatory RNAs long of 20-24 nucleotides that post-transcriptionally regulate gene expression by directing mRNA cleavage or by translational inhibition. MicroRNA are generated from a single strand of RNA precursor that forms partially double stranded stem-loop structures (hairpins) through intramolecular base pairing. Typically, these endogenous RNAs are each transcribed as a long RNA and then processed to a pre-miRNA of approximately 60-75 nucleotides that forms an imperfect hairpin (stem-loop) structure. The pre-miRNA is then cleaved to form the mature miRNA.

MicroRNA repress gene expression by guiding effector complexes (miRNPs or RISC) to complementary sites on mRNAs (Bartel, 2004). Because of the extensive sequence complementarity between plant miRNAs and their target mRNAs, RISC recruitment in plants typically leads to target mRNA cleavage (Carrington and Ambros, 2003; Bartel, 2004; Schwab et al., 2005).

According to the present invention, the term "a DNA sequence coding for a microRNA" means that at least the precursor form of the microRNA, called pre-Sly-mRNA, is transcribed; in particular, said microRNA is over-expressed using a constitutive promoter. The DNA sequence coding for the microRNA can be oriented "sens" or "anti-sens". The over-expression of an anti-sens construct of the pre-Sly-mRNA results in a decrease in the amount of mature form of the endogenous microRNA, since the anti-sens construct will hydridize with the endogenous microRNA. On the contrary, the ectopic expression of a sens construct results in an increased accumulation of the corresponding miRNA.

The term "post transcriptional regulation", as used herein, refers to regulation of protein expression after the gene for the protein has been transcribed into an mRNA.

The term "decreases the expression level of said ARFs" designates the fact that the expression of the ARF, compared to a wild-type plant, is about 20% less, preferably about 30% less, 40% less, and more preferably is about 50% of the usual level of expression.

The man skilled in the art knows how to measure the level of expression of a protein, with widely used techniques such as protein quantification and western blot.

The present invention is related to a flowering plant expressing a DNA sequence coding for a microRNA, wherein the microRNA is specific to members of the Auxin-Reponse Factor (ARF) gene family and decreases the expression level of said ARF when expressed.

It is understood that the invention concerns any type of flowering plant, in particular plants selected among fruiting and flowering vegetables, including Armenian cucumber (Cucumis melo Flexuosus group), Eggplant (Solanum melongena), Avocado (Persea americana), Bell pepper (Capsicum annuum), Bitter melon (Momordica charantia), Caigua (Cyclanthera pedata), Cape Gooseberry (Physalis peruviana), Cayenne pepper (Capsicum frutescens), Chayote (Sechium edule), Chili pepper (Capsicum annuum Longum group), Cucumber (Cucumis sativus), Globe Artichoke (Cynara scolymus), Luffa (Luffa acutangula, Luffa aegyptiaca), Malabar gourd (Cucurbita ficifolia), Parwal (Trichosanthes dioica), Perennial cucumber (Coccinia grandis), Pumpkin (Cucurbita maxima, Cucurbita pepo), Pattypan squash Snake gourd (Trichosanthes cucumerina), Squash (Cucurbita pepo), Sweetcorn (Zea mays), Sweet pepper (Capsicum annuum Grossum group), Tinda (Praecitrullus fistulosus), Tomato (Solanum lycopersicum), Tomatillo (Physalis philadelphica), Winter melon (Benincasa hispida), West Indian gherkin (Cucumis anguria), Zucchini (Cucurbita pepo), and more preferably tomato plants.

In a preferred embodiment of the invention, the plant belongs to families of Solanaceae or Cucurbitaceae.

In a more preferred embodiment of the invention, the plant is chosen among: *a Solanum lycopersicum, a Capicum a Capsicum annuum, a Solanum tuberosum a Solanum melongena, a Cucumis melo, a Cucumis sativus, a Cucurbita pepo* or a *Citrullus lanatus.*

In a specific embodiment of the invention, the specific microRNA is expressed under the control of an ubiquitous promoter, such as a 35S promoter. Therefore, this microRNa is constantly expressed in the plant, and constantly inhibits the expression of specific members of the ARF family.

In an embodiment of the invention, the target ARF whose expression is decreased is a functional equivalent of ARF2, ARF3, ARF4, ARF7, ARF10 or ARF17 from *Solanum lycopersicum.*

A 'functional equivalent' designates a protein having the same activity of said ARF, even if not the exact same polypeptidic sequence. Methods for determination of the percentage of identity between two protein sequences are known from the man skilled in the art. For example, it can be made after alignment of the sequences by using the software CLUSTALW available on the website http://www.ebi.ac.uk/clustalw/ with the default parameters indicated on the website. From the alignment, calculation of the percentage of identity can be made easily by recording the number of identical residues at the same position compared to the total number of residues. Alternatively, automatic calculation can be made by using for example the BLAST programs available on the website http://www.ncbi.nlm.nih.goy/BLAST/ with the default parameters indicated on the website.

It is well known in the art that a polypeptide can be modified by substitution, insertion, deletion and/or addition of one or more amino-acids while retaining its enzymatic activity. For example, substitutions of one amino-acid at a given position by a chemically equivalent amino-acid that do not affect the functional properties of a protein are common. For the purposes of the present invention, substitutions are defined as exchanges within one of the following groups :
■ Small aliphatic, non-polar or slightly polar residues : Ala, Ser, Thr, Pro, Gly
■ Polar, negatively charged residues and their amides : Asp, Asn, Glu, Gln
■ Polar, positively charged residues : His, Arg, Lys
■ Large aliphatic, non-polar residues : Met, Leu, Ile, Val, Cys
■ Large aromatic residues : Phe, Tyr, Trp.

Thus, changes which result in substitution of one negatively charged residue for another (such as glutamic acid for aspartic acid) or one positively charged residue for another (such as lysine for arginine) can be expected to produce a functionally equivalent product.

The positions where the amino-acids are modified and the number of amino-acids subject to modification in the amino-acid sequence are not particularly limited. The man skilled in the art is able to recognize the modifications that can be introduced without affecting the activity of the protein. For example, modifications in the N- or C-terminal portion of a protein would not be expected to alter the activity of a protein.

In a specific embodiment of the invention, the target ARF whose expression is decreased into the flowering plant has a nucleotidic sequence having at least 80% of identity with at least one sequence chosen amon the following sequences: SEQ ID N°1 (ARF10), SEQ ID N°2 (ARF17), SEQ ID N°3 (ARF2), SEQ ID N°4 (ARF3), SEQ ID N°5 (ARF4), SEQ ID N°6 (ARF7). Methods for determination of the percentage of identity between two nucleotidic sequences are known from the man skilled in the art. Advantageously, the identity percentage between two sequences is calculated by the global alignment algorithm developed by Needleman-Wunsch. (Needleman SB, Wunsch CD. "A general method applicable to the search for similarities in the amino acid sequence of two proteins". J Mol Biol. 1970 Mar;48(3):443-53).

In a specific aspect of the invention, the mature form of the microRNA expressed in the plant has a sequence presenting at least 80% of identity with the sequence as shown in SEQ ID NO 7.

In a prefered aspect of the invention, the specific microRNA is the "miR160" isolated from *Solanum lycopersicum,* presenting the sequence as shown in SEQ ID N0 7.

The present invention is also related to a microRNA whose mature form has a sequence presenting at least 80% of identity with the sequence as shown in SEQ ID N0 7.

The present invention is also related to a specific microRNA called "miR160", isolated from *Solanum lycopersicum,* presenting the sequence as shown in SEQ ID N0 7.

The invention is also related to a plant transformation vector comprising a microRNA such as defined above, under the control of a promoter functional in plant cell.

The invention is also related to a plant cell transformed with a vector such as defined above.

The invention is also related to a parthenocarpic flowering plant, comprising at least one plant cell transformed with a vector such as defined above.

The invention also concerns a method for producing a parthenocarpic flowering plant, comprising the steps of :
● providing a plant cell;
● transforming the plant cell with a transformation vector such as defined above;
● regenerating and growing the transformed plant cell to obtain a transformed plant,
● selecting a fertile transformed plant.

Preferred phenotypes of flowering plants and particularly of tomato plants according to the invention, are presented below.

### DRAWINGS

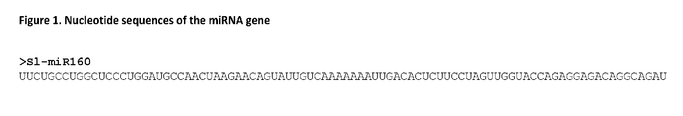

**Figure 1****.** Nucleotide sequences corresponding to Sl-miR160 gene isolated from tomato (*Solanum lycopersicum*)*.* The Sl-microRNA gene encodes small interfering RNAs that repress the expression of the ARF genes by targeting their transcripts for degradation. The gene product of Sl-miR160 regulates the expression of Sl-ARF10 and Sl-ARF17 genes at the post-transcriptional level.

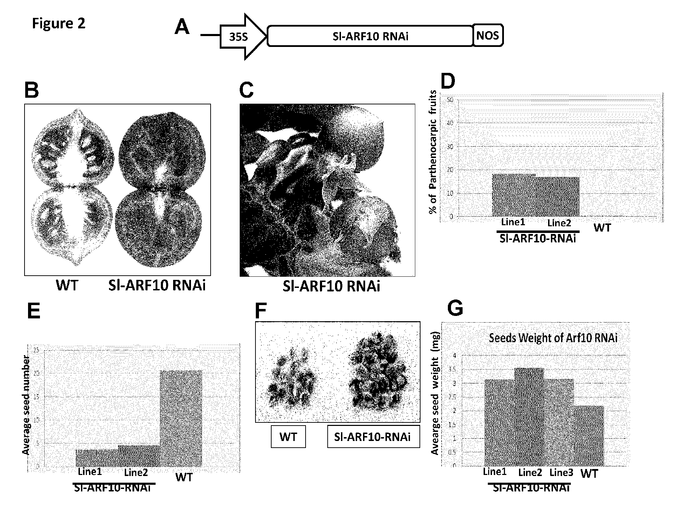

**Figure 2****.** Down-regulation of Sl-ARF10 results in parthenocarpic fruit and increased seed size and weight.
   **(A)** The RNAi construct used to down-regulate the expression of Sl-ARF10 in the tomato.
   **(B)** Down-regulation of Sl-ARF10 results in parthenocarpic fruit.
   **(C)** Down-regulation of Sl-ARF10 results in precocious fruit set prior to pollination.
   **(D)** Up to 17-18% of the fruit produced by the Sl-ARF10 down-regulated lines are totally seedless.
   **(E)** The mean seed number per fruit is strongly reduced in the Sl-ARF10 down-regulated lines.
   **(F)** The seed size is significantly bigger in the Sl-ARF10-suppressed lines.
   **(G)** The seed weight is increased by 41 to 60% in the Sl-ARF10 down-regulated lines.

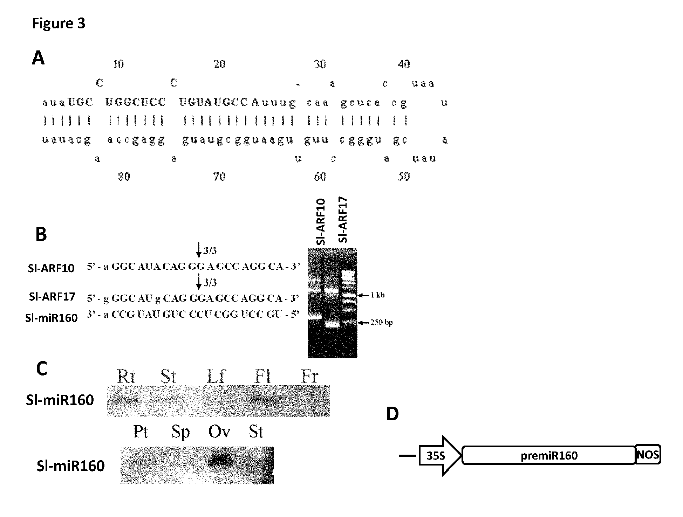

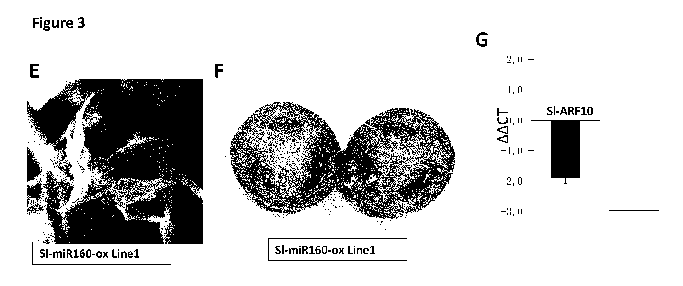

**Figure 3****.** Sl-ARF10 expression is regulated at the post-transcriptional level by SI-miR160 and the ectopic expression of Sl-miR160 in the tomato results in parthenocarpic fruit.
   **(A)** The nucleotide sequence of the pre-miR160 predicted to give rise to a hairpin structure required for miRNA activity.
   **(B)** Location of the single cleavage site within Sl-ARF10 and Sl-ARF17 transcripts that are the targets of the Sl-miR160. The figures associated with the arrows indicate the prevalence of the cleavage forms. The experimental evidence for the presence of the mRNA cleavage products of Sl-ARF10 and Sl-ARF17 is provided by RNA gel analysis.
   **(C)** Expression pattern of the tomato Sl-miR160 gene in different plant tissues and organs. Of particular interest, the expression of the Sl-miR160 gene is extremely high in the ovary.
   **(D)** The Sl-miR160 sense construct used for over-expression of the Sl-miR160 gene in the tomato.
   **(E)** Ectopic expression of Sl-miR160 results in precocious fruit set prior to flower pollination.
   **(F)** Over-expression of Sl-miR160 in the tomato results in parthenocarpic fruit.
   **(G)** Reduced accumulation of Sl-ARF10 transcripts the tomato lines over-expressing the Sl-miR160 gene.

### EXAMPLES

### Example 1 - Identification of microRNA in tomato

In silico search for potential microRNA-mediated cleavage sites in the MirBase database allowed the identification of sequences specific to ARF10 and ARF17: miR160.

The functionality of the identified sites was validated through the isolation of the mRNA-cleaved forms corresponding to the ARF target genes using rapid amplification of cDNA ends (RACE) (Llave et al., 2002). The MIReNA program (Mathelier et al., 2010) was then used to identify the miRNA genes in the tomato genome sequence. The expression of the precursor forms of the miRNAs has been validated by RT-PCR and the corresponding sequences cloned in the appropriate expression vector for functional studies.

### Example 2 - Post-transcriptional regulation of ARF10 with Sl-miR160

Tomato plants are transformed with expression vectors expressing Sl-miR160 (SEQ ID N°7) under control of an ubiquitous promoter (construction is shown on figure 3D). The ectopic expression of Sl-miR160 results in the inhibition of the expression of ARF 10 in transgenic tomato lines (Fig 3G), in precocious fruit set prior to flower pollination (fig. 3E) and in parthenocarpic fruits (Fig. 3F).

### REFERENCES

● Wang H, Schauer N, Usadel B, Frasse P, Zouine M, Hernould M, Latché A, Pech JC, Fernie AR, Bouzayen M. "Regulatory features underlying pollination-dependent and - independent tomato fruit set revealed by transcript and primary metabolite profiling." Plant Cell. 2009 May;21(5):1428-52. Epub 2009 May 12*.*
● Gorguet B, Eggink PM, Ocaña J, Tiwari A, Schipper D, Finkers R, Visser RG, van Heusden AW. "Mapping and characterization of novel parthenocarpy QTLs in tomato." Theor Appl Genet. 2008 Apr;116(6):755-67. Epub 2008 Jan 30*.*
● de Jong M, Wolters-Arts M, Feron R, Mariani C, Vriezen WH "The Solanum lycopersicum auxin response factor 7 (S1ARF7) regulates auxin signaling during tomato fruit set and development." Plant J. 2009 Jan;57(1):160-70. Epub 2008 Oct 4*.*
● de Jong M, Wolters-Arts M, Garcia-Martinez JL, Mariani C, Vriezen WH. « The Solanum lycopersicum AUXIN RESPONSE FACTOR 7 (SlARF7) mediates cross-talk between auxin and gibberellin signalling during tomato fruit set and development. » J Exp Bot. 2011 Jan; 62(2): 617-26. Epub 2010 Oct 11*.*
● Dalmay T. "Short RNAs in tomato. » J Integr Plant Biol. 2010 Apr; 52(4):388-92*.*
● Guilfoyle et al. (1998) Plant Physiol. 1 18:341 -347*.*
● Kim et al. (1997) Proc. Natl. Acad. Sci. USA 94: 1 1786-1 1791*.*
● Ulmasov et al. (1997a) Science 276: 1865-1868*.*
● Ulmasov et al. (1997b) Plant Cell 9: 1963-1971*.*
● Ulmasov et al. (1999) Plant J. 19:309-319*.*
● Bartel DP. "MicroRNAs: genomics, biogenesis, mechanism, and function." Cell. 2004 Jan 23;116(2):281-97*.*
● Carrington JC, Ambros V. "Role of microRNAs in plant and animal development. » Science. 2003 Jul 18; 301 (5631): 336-8*.*
● Schwab R, Palatnik JF, Riester M, Schommer C, Schmid M, Weigel D. "Specific effects of microRNAs on the plant transcriptome." Dev Cell. 2005 Apr;8(4):517-27*.*
● Needleman SB, Wunsch CD. "A general method applicable to the search for similarities in the amino acid sequence of two proteins". J Mol Biol. 1970 Mar;48(3):443-53
● Llave, C., Xie, Z., Kasschau, K.D., and Carrington, J.C. (2002). "Cleavage of Scarecrow-like mRNA targets directed by a class of Arabidopsis miRNA." Science 297, 2053-2056*.*
● Mathelier A, Carbone A (2010). "MIReNA: finding microRNAs with high accuracy and no learning at genome scale and from deep sequencing data". Bioinformatics. 15;26(18):2226 34*.*

## Claims

1. A flowering plant expressing a DNA sequence coding for a microRNA, wherein the microRNA is specific to members of the Auxin-Reponse Factor (ARF) gene family and decreases the expression level of said ARFs when expressed.

2. The plant of claim 1 wherein the specific microRNA is expressed under the control of an ubiquitous promoter.

3. The plant of claim 1 wherein the target ARF whose expression is decreased is a functional equivalent of ARF2, ARF3, ARF4, ARF6, ARF7, ARF10 or ARF17 from *Solanum lycopersicum.*

4. The plant according to anyone of claims 1 to 3, wherein the target ARF has a nucleotidic sequence having at least 80% of identity with at least one sequence chosen amon the following sequences: SEQ ID N°1 (ARF10), SEQ ID N°2 (ARF17), SEQ ID N°3 (ARF2), SEQ ID N°4 (ARF3), SEQ ID N°5 (ARF4), SEQ ID N°6 (ARF7).

5. The plant according to anyone of claims 1 to 4, wherein the mature form of microRNA has a sequence presenting at least 80% of identity with the sequence as shown in SEQ ID N0 7.

6. The plant according to anyone of claims 1 to 5 wherein the specific microRNA is the "miR160" isolated from *Solanum lycopersicum,* having the sequence SEQ ID NO 7.

7. The plant according to anyone of claims 1 to 6, wherein the plant is a Solanaceae or a cucurbitaceae.

8. The plant according to anyone of claims 1 to 7, wherein the plant is chosen among : *a Solanum lycopersicum, a Capsicum annuum, a Solanum tuberosum a Solanum melongena, a Cucumis melo, a Cucumis sativus, a Cucurbita pepo, or a Citrullus lanatus.*

9. A microRNA having the sequence as defined in claims 5 or 6.

10. A plant transformation vector comprising a microRNA according to claim 9, under the control of a promoter functional in plant cell.

11. A plant cell transformed with a vector according to claim 10.

12. A parthenocarpic flowering plant, comprising at least one plant cell according to claim 11.

13. A method for producing a parthenocarpic flowering plant, comprising the steps of :
● providing a plant cell;
● transforming the plant cell with a transformation vector according to claim 11;
● regenerating and growing the transformed plant cell to obtain a transformed plant,
₀ selecting a fertile transformed plant.


SEQUENCE LISTING

<110>  INP TOULOUSE
       Bouzayen, Mondher
       Zouine, Mohamed
       Pech, Jean-Claude
       Latche, Alain

<120>  NEW PARTHENOCARPIC PLANTS WITH MODIFIED EXPRESSION OF AUXIN
       RESPONSE FACTORS AND THE MICRO-RNAs INDUCING SAID MODIFIED
       EXPRESSSION

<130>  69245 D29651

<160>  7

<170>  PatentIn version 3.5

<210>  1
<211>  2100
<212>  DNA
<213>  solanum lycopersicum

<400>  1
atgaaggagg ttttggagaa gtgtgtggat tcacagctat ggcacgcctg tgcaggagga      60

atggtgcaaa tccctccggt taactccaaa gtctactatt ttccacaagg gcatgctgag     120

catactctta tgaatgtgga tttctcagct ctaccaagaa gtcctgctct gattctatgc     180

agagtagctg ctgtgaagtt cttagcggac cctgaaacag atgaggttta tgctaagatt     240

agggttgtcc cagttgggaa caaggggaat gactttgatg atgatgatga cattttgggt     300

agtaatgagt caggaacagc tgaaaaaccc aattcatttg ccaagacatt gactcaatca     360

gatgcaaata atggtggtgg attctctgtg cctagatact gtgcagagac aatatttcct     420

aggttggatt acacggctga cccgcctgtg cagaccgtga cagccaaaga tgttcatggt     480

gaaagttgga agtttaggca catttataga ggtactccca ggaggcattt gttaacgact     540

ggttggagta gttttgtgaa tcagaagaaa cttgttgctg gagattccat tgtgtttttg     600

agggcagaaa atggtgaact ttgtgttgga attcgtaggg cgaaaagagg tggtattggt     660

gggcctgaag ccccatctgg atggaactct ggtgctggaa attatggtgg attttctgcg     720

tttttgaggg aagagatgag caaaaatgga aatttgactt ctcctactag gagcttaagg     780

ggaaagggaa gggtgaggcc tgaatcagtt gttgaagctg catatcttgc ttctagtggg     840

cagccttttg aagttgttta ttatccccgt gcaaacacac cagaattttg tgttagggca     900

tcttcagtga atgctgcaat gaggattcaa tggtgctcag gaatgaggtt caaaatggct     960

tttgaaactg aggattcttc tcgaatcagt tggttcatgg gaactatatc ctccattcaa    1020

cttgctgatc ccatccgctg gcccaattcg ccatggaggc ttcttcaggt ggcatgggat    1080

gaacctgatt tattgcaaaa cgtaaaacat gtcagcccat ggcttgtcga attggtctca 1140

aatatgccag tcattcacct ctcgcccttc tcaccgccaa gaaaaaagct gcgtctacca 1200

ccagatttct cacttgacag ccagtttcag ttaccatctt tttcaggcaa ccccctcagg 1260

tccagcagtc ctttctgttg tttatctgac aacatcactg caggcataca gggagccagg 1320

catgctcaat ttggagtacc tttattggat cttcacctta gcaacaaatt accgtcagga 1380

ctgttaccac caagtttcca gcgcgttgca gccaactcac aacttcctaa tgtcatcaat 1440

aagtgccaaa atgacagaaa tgataacatc tcttgtttgc ttacaatggg tacttcaagt 1500

aagacattgg acaaaaatga tagtgtgaat acacctcggt tcttactctt cggtcagcca 1560

attctgactg agcaacaaat ctctaatgga tgttctgtca gtgctccaca agtagtccaa 1620

acagggaaag acttaggaag gatacaaccg atcaacgaaa aacatccttc tgagcagaaa 1680

ggcagcattc aagacaatct atcaagtgca acatttttct ggaatcgagg ttatcatgca 1740

gctgaacttg gtgtactcaa tactggtcac tgcaaagtat tcctcgagtc agaagatgtt 1800

ggccgtacac ttgatctgtc agttatggga tcatatgaag aactgtacaa aagacttgca 1860

aacatgtttg gacttgaaag accagatatg ctgacccgtg tgctctatca tgatgcaaca 1920

ggtgctgtta aacacaccgg agatgaacca ttcagtgact ttgttaagag tgcaaaaaga 1980

ttgacaattc tgatgaactc aagcagcaat attaaaagga aatggttaac tggtctcgca 2040

actgctgaac gtggtctaga ttcatcaaac caggcaggac ctcttagcat ctttgcatag 2100

<210> 2
<211> 1869
<212> DNA
<213> solanum lycopersicum

<400> 2
atgcaggctc caaccgtcgc cgaccgtgaa gtggacccta tagtctggcg agcaatcgcc 60

ggaaactctg tcaaaatacc tcccgtcggt accagggttt actactttcc tcaaggacac 120

gctgaacatg caacctttac ttcacctgct gtaatgtctc ctggtatgcc tgcgtttatt 180

ctatgccgtg tgttatctgt tcgttttctc gctgaatccg atacggacga ggtatacgct 240

aggatttttc tgcatccgat tagtcagagt gaagttgatg aagttactat gagggaggag 300

gaggtggttg aggacgaaat cgtgtcgttt gtgaagattt taactccgtc agatgcgaat 360

aatggaggag gtttctctgt gccgaggttc tgtgctgatt ctatatatcc tcggcttgat 420

tttgaggcag aaccgccggt tcagaatttg tctattaggg atattaaagg tgttgcgtgg 480

gagtttcgac acatttatcg tggaacacct cgacggcatt tgcttactac tggttggagc 540

aagtttgtta acagtaaaca gcttgtcgct ggtgattccg ctgtgttcat gcggagaact 600

gcaaataatc aactctatgt gggtgtccga cgagctataa gacgaaatga cgattcgcag 660

aagtggactt cctccttttt gatgcgtgaa catatcaata atggtggtag cccggatgtg 720

tcttggggga ttagaaaggg gcggatgacg atggaggctg ttgcggctgt agcggaaaaa 780

gcggcacggg gagtgccatt tgaagtatcg tgttatccac gggatgcctg ggcaggtttt 840

gtggtgaagg cacaagaggt acagatggca ttgaatatgc cttggacagt tggcatgagg 900

gttaagatgg ctgtggaagc ggaggattca tctagaacgg cttgctatca agggactgtt 960

tcatctgtca ttttgaatga aagtggccct tggcgtggat ctccatggcg aatgcttcag 1020

atcacttggg aggaaccaga agttccgcag catgctaaca gggtgaatcc ttggcaagtt 1080

gagtgttttc cgccaatccc acagtttctt cctccatcga agaaaataaa actgcctaat 1140

gggcttctac ctgatggaga gagaagcccc tttcctatga cagggttggg tagcttccct 1200

atgacagggt tgggtaactt tcctatgaca gggttgggta acttccctat gacagggttg 1260

ggtagcttcc ctatgacagg attgggtagc ttccatatga cagggttggg tagcttccct 1320

atgacagggt tgggtaattc tacgattgga ctcagtagcc catcattggg caattttaca 1380

tcttttcctg cgggcatgca gggagccagg catgatcaag ttagtgtatc aagtttatcc 1440

aatgttaaga gcaataacct tgggttgtgc actaacaata gcttagatga agaaatcaag 1500

gcaaagcttg acagtgtttc tcccaaactg aatattggca gttcatattc agacaattta 1560

tcactggata gtcagagcag tgtgcatttt ggggacaatg agctgattac aaaaccgggt 1620

tccagctctt ttacaaaaga tcgtttcagt actttccagt tatttggtaa ggtcatccat 1680

gttgaacgtg ttgagggagc tttagatggt tttggtttct ctgaaagtga taacgttgaa 1740

gtgtataaag agatagatga tccatccaac tcagacgtct caccaaataa agatgatcca 1800

tccaactcag atgtctcacc aaataaacct ttcaagctgt ttgacaatcc tgatgtccaa 1860

catgaatga 1869

<210> 3
<211> 2490
<212> DNA
<213> solanum lycopersicum

<400> 3
atggctactt ctgagaattg ccggaacgct gccggcgccg gaaaagttga tgctgagaaa 60

gcgttgtaca cggagctatg gcgagcatgt gcaggtccgc ttgtgacagt gccatgtgaa 120

ggcgagctgg tgttctattt cccacaaggt catattgaac aggttgaagc atcaacaaac 180

caagcttcag accagcagat gccagtatat aatcttcctt ctaagatcct ctgtcgtgtg 240

attaacgtcc tgctgaaggc tgaacccgat acagatgagg tgtatgcaca agtgactttg 300

ttgccagaac caaatcaaga tgagaatgtg gtatcaaagg aaccgatgcc ctctccgcca 360

ccacgattcc atgtgcactc tttttgtaag acattaacag cctctgatac cagcactcat 420

ggaggatttt ctgtcttgag acggcatgct gatgaatgcc tcccgcctct ggatatgtct 480

cggcagcctc caacacagga gttggtggcc aaagatttgc atgcaaatga gtggcgcttc 540

aggcacatat tccgggccca gcctaggagg caccttcttc agagtggttg gagtgtcttt 600

gttagttcga aaaggcttgt tgcaggggat gcattcatat ttcttagagg tgagaatggg 660

gagcttcgtg ttggagtccg acgtgccatg agacagcagg gtaatgctcc atcatcagtg 720

atatccagtc atagcatgca tcttggtgtc cttgctacag cttggcatgc tattcagacg 780

aaaacactgt tcacagtata ttataaacca aggacgagcc ctgctgactt tatagttcca 840

tatgatcagt atatggagtc tctgaaaaac aattactcca tcgggatgag gtttaaaatg 900

aggtttgaag gtgaagaagc tccagaacag aggtttactg gaactatagt tggcattgaa 960

aatgctgacc tcaaaaggtg gcctgagtca aaatggagat gcctgaaggt tcgatgggat 1020

gaaacttctg ctattcctag gccagaccga gtttcaccct ggaaagtaga gccagctctt 1080

agccctcctg cacttaatcc acttccaata ccaaggcaga aaaggccgcg atcaaatgtt 1140

ctgccctcgt ctcctgattc ttctgtactt actagggaag gttcatccaa agtagttgta 1200

gacacttcac aagccagtgg gttttcaaga gttttgcaag gtcaagaaat atcaaccttg 1260

agaggcaatt ttgtagaaaa taacgagtcg gactcttctg agaagccacc tatatggcaa 1320

ccattactgg atgacgagaa ggctgatgtt cattctgcgt caaggaaatg tatatcagat 1380

aaacggcttc ctttagggag gcctgaatca tcttttacag atcttttatc aggttttggg 1440

gggcaatcta gttcatctca tggattccat tcacccactg ggggccaaac agcacctgct 1500

agctgggtta agcgacaagc tctggataag gaaactgatt tcagcttact ggcaaaacaa 1560

tggtctctag tgtcttctgg tctctcactt aatctgatgg aatcaggatt gaagggtgca 1620

gatactctgt atcaaatgcg gggaacatct cgactcaatt gttttaacga gtatccaacc 1680

ttccctggtc atagacctga caatcagcag ggaaattggt taatgccccc gtccgtgctg 1740

ccttatattc agatgtccgc tcattctgga gaaattatgc ctaaacccat ggcttcacca 1800

cagcccgaag ccatgaaacc caaagaaggg aactgcaaac tatttggcat tccccttgta 1860

agtaaatgtg ccaccataga tcctgtcatg ttgcggaaaa attccccgat tcactcaaca 1920

agtaacatgc actttggtat acatccacat caattcccta taattgaatc tgatcaaagg 1980

tctgagcaat caaagggatc aaagctacca gatgatggct tcatagttca tgatcaggaa 2040

gaacaattcc aaacctctca tcctggtact cgagatagag agggcaaagg ccttgttcat 2100

tcaacaagga gttgcaccaa ggttcataaa cagggtacag cccttggaag gtctgttgat 2160

cttgcaaagt tcaacaacta tgaagaattg atagctgaac tggatcacat tttgattttt 2220

aatggtgagc tcaaggctcg taacaagaac tggctggttg tatatactga tgatgagggt 2280

gacatgatgc ttgttggaga tgatccatgg caggaatttt gtggtatggt tcggaagatt 2340

tttatctaca cgaaagatga ggtgcagcgg atgaaccctg ggactctcaa ttcaaaaggc 2400

gaggacaatt cttctgttgc agaaggctct gatgctaaag aagtgaagaa tctacagctt 2460

cacattgatt ccagtccgga agattcttag 2490

<210> 4
<211> 2244
<212> DNA
<213> solanum lycopersicum

<400> 4
atgatgtgtg gacttattga tctgaatact gtggataacg atgatgccgg agaagaaacg 60

acggcgccgg tgtcattgga ttcaccggcg tcgtcgtcgg cggcatcagg aagttcggat 120

ttaacgtcgt caactacgcc agcggtggca tcggtgtgta tggagctttg gcatgcgtgt 180

gctggaccgt tgatttcgct gccgaagaaa ggaagtgcgg ttgtgtacct gcctcaaggt 240

cacttggaac atttatctga gtacccgtcc atagcctgta atctccctcc tcatgtgttt 300

tgtcgcgttg tagatgtgaa gctacaagca gatgcggcta ctgatgaggt ctatgcacaa 360

gtctcactag ttcctgacaa tcagcagatt gagcagaaat ggaaggatgg agacattgat 420

gctgatattg aagaagagga aatagaaggt gctggaaaat caataacacc acacatgttc 480

tgcaaaactc ttactgcatc ggataccagc actcatggtg gtttctctgt ccctcggcgg 540

gcagcagaag attgttttgc tcccttggat tacagacaac agaggccctc gcaggagctg 600

gtagccaaag atctacatgg tatagagtgg aaatttcggc atatctatcg gggtcagcca 660

cggcggcatc tgctcactac aggatggagt gcatttgtaa acaagaagaa gcttgtttct 720

ggtgacgctg ttcttttctt gaggactggt gatggagagc ttaggttagg agtgagacga 780

gctgcccaag caaaaacatg ttctagttat ctggctcctt gtagcaaacc gttgaatgtt 840

agtggcattg tagatgctgt taacgttata tctagcagaa atgctttcaa catttgttac 900

aacccaaggg atagctcatc ggatttcatt gtaccttacc acaaattctc taagactctt 960

gcacatccct tttcagctgg aatgaggttt aaaatgcgtg tcgaaacaga agatgcagct 1020

gagcaaaggt tcactggact agttgtggga gtcagcaatg tagatccagt tcgatggcca 1080

ggttctaaat ggaggtgcct attggtcaga tgggatgatc ttgatgtttc tagacataat 1140

agggtttcac catgggagat tgagccatct ggttcagctc ctgtgcccag cagcttggtg 1200

atgccttctg ctaagaggac cagggttggc ttccctattt caaaggcaga ttttccaatt 1260

cctagagaag gaattgcagt atcagacttt ggggaacctt ctaggttcca gaaggtcttg 1320

caaggtcaag aaattttgag gatgcatgct ccttatggcg gacttgatgc tcggagtcct 1380

cgtccagcag gcacaagatg ctttcctggt tttcctagtt ctgggatatc tagaatggga 1440

aacagcatca gacccctgtt tggtgacact gacaagtccc atgaaagcat tggctttagt 1500

gaatctcttc gattcaataa ggtcttgcaa ggtcaagaaa tttttacaag ccctccttat 1560

gggagagctc aagctggtat ccaaatgcag gagaaaagta ggaccggtat ttttgtcggt 1620

attcaggttc caaaccatgg aaacaggtgg cctgctccaa atcaggataa taacactcct 1680

tgcaagccaa ttaatcctgt ctcagcatca tcaccgcctt ctgcactcaa ttttcagcat 1740

ccgagccctc cagcatcaaa gttccaggct atgttcaatc ataaacatga tcttgttaac 1800

caggcttcgt tagatctgtc tgagaactgt tgtaggtatc cgtatctctc atctggttca 1860

cataccgagg acatcagtca gaaggaaggt actcaaggaa tcagctcgtt tggtttctta 1920

aaggagcaaa agcaaacagg actttcatat ctttctcctg ggacacagtc gtcattcaaa 1980

ggcaatcaaa acttagtttc cacttgtaaa actggttgca ggatctttgg gttccccttg 2040

accgagagta aaataagtgc aactagagcg gatactccct ctgaagctgt atactcacat 2100

ggtctagaaa ctacatttct cccttccagt gatggaaagt tgcagccggg gccaccattg 2160

atgactaacg ttgtggggac aaattttacc aaagtaaatg acctctatgc agcaagagat 2220

gtgcttcttg atattgctct gtag 2244

<210> 5
<211> 2436
<212> DNA
<213> solanum lycopersicum

<400> 5
atggaaattg atctgaatca tgcattagtg agtgaagtgg agaagaatgt atgttgtaat 60

gaagaatgtg ataaaggggg tggtgggggt tgtgttaatt gctctttgta cacttccact 120

acttcttctt gttcttcaaa tgtatcttca tcttcttcac ttgctcttac ctcaatttat 180

aaagagcttt ggcatgcttg tgctggtcct cttactagtt tacccaagaa aggaaatgta 240

gtggtttatt tccctcaagg tcatatggaa gaagctgttt ctgcttttcc attttcacct 300

gtcaagattg atttaccgac ttttggtctt cagcctcaga tcttctgtag agtggaagat 360

gtccaattgc ttgcaaataa ggaaaatgat gaggtgtata cacagctcac tcttcttcct 420

cttccagagt caatggctat aagcttagag gggaaagaac atgaagattc ggggacggat 480

gaggaaggga atggagtcaa tccagggaaa tcagcttctc acatgttctg taaaacgctg 540

actgcttcgg ataccaccac ccatggtggc ttttcagttc cccgtcgagc agctgaagac 600

tgcttccccc ctctggatta caaagagcaa agaccctcac aggagctaat tgctaaggat 660

ctgcacggag tagagtggaa atttcgtcac atttatagag gccagccaag gcgacatttg 720

ctcactactg gatggagtat ctttgtcagc caaaagaatc ttgtctcagg ggatgcagtt 780

ctcttcttga ggggagaagg tggaaatctc agactgggaa taagaagagc tgcaagacct 840

agaaatgggc ttcctgaatc gataatcaaa agtcaatatt ctggaccgga tgttctttcg 900

tcagttgcta cagccctatc agcaaagagc actttccatg ttttctatag cccaagggca 960

agtcatgctg atttcgttgt accttaccaa aagtacgtga aagccatcaa ctctcggatt 1020

cctgttggga caagatttaa aatgaaattt gatttggatg attcacctga aagaaggtac 1080

agcggtgtag tgactgggat aagcgatatg gatcccttta gatggcccaa ctctaaatgg 1140

agatgcttga tggtcagatg ggatgaagat ataatgagta atcatcagga acgggtttct 1200

ccttgggaaa ttgattcttc agtttcctta ccaccactga gcattcagtc ctcaccaaga 1260

ctgaagaaac tgcggactag tcagcaggca ccatcagtac tggacagcca ttttgctgga 1320

gggagtgctc ttttagactt tgaggagtca ataagatcct ccaaggtctt gcaaggtcaa 1380

gaaaatttag gtctgatatc acctccctat ggttgtgata aaccagtccg cccactggat 1440

ttcgagctgc aaagagtagc tcgtcacaat ctcatgccaa atggtgtaga gaacattatt 1500

gttggtgact ttgtgaaaac tcagcctcca acaacctaca caggctttct ggaatccaat 1560

aggtttccaa aggtcttgca aggtcaagaa atttgctcgt tgagatccct tactggaaaa 1620

ggtgatgtta attttggtgc ttggggaaaa cctgaatttg gttgcaatgt tttcggcaca 1680

tatcagaggc cgagagccaa tttctatcct ctcgcttctg aaggggcgag gaatgtgttt 1740

ttaccttaca atgcgatgta tagagctgga caagatcccg tggttccctc ctacagtact 1800

aatttccaaa gagaaaatcc taccttgaac cagaattcaa tccaaaatgt ggttaggagg 1860

gaagaagttg gaatgccaaa gtttgtaaat gagcagaggc caccggagat gtctaaagtg 1920

tccattccag agaaccattt caagaatgaa aatgacgact ccttcaatgc acaggctcca 1980

tgtaaactgt ttggcttttc cttgacgaaa gaaccatcta ctcctagctc gcagagttct 2040

ggtaagagga gctgtactaa ggttcacaaa caaggcagct tggttggacg agccattgat 2100

ctctcgagat tgaatggcta tgacgacttg ctggttgagt tggagaggct tttcaacatg 2160

gaagacctct taagagatcc caacaagggg tggcgaatct tgtacactga cagtgagaat 2220

gacatgatgg ttgtcggaga tgatccatgg catgagttct gtgaggtagt atccaagatc 2280

catatataca ctcaagaaga agtggagaaa atgactattg aggggatcag cgatgacact 2340

caaagttgtt tggaggaggc accagcaatc atggatgtct caaagtcttc ttcagttggc 2400

cagcctgatt catctccaac tgtaatcagg atttga 2436

<210> 6
<211> 3339
<212> DNA
<213> solanum lycopersicum

<400> 6
atgaaaacac cggtgaatac tgccggagtt cagcagcagc atactgttaa cgggaatcct 60

ggtgaagtgg agaagaaaag cataaatcca gagctatggc aggcctgcgc cggtcctctg 120

gtgaacttac cggcagctgg gacccacgtt gtttattttc cacaaggcca cagtgaacag 180

gttgcggcat ctatgaagaa agatgtagat gcccaaatac caaactatcc aaaccttcca 240

tcaaagttag tttgtctcct tcacaatatt accctgcatg cggatccaga aacagatgaa 300

gtttatgcac aaatgacact gcagccagtt ccttcatttg ataaggaggc attgttgaga 360

tcagatttgt ctatgaaagc aaataaacca caaacagaat ttttttgcaa gaccttgact 420

gccagtgata caagcacaca tgggggtttt tccgtccctc gacgttctgc agaaaaaata 480

ttcccgcctc tggattactc aatgcaacca cctgctcaag aacttgtggc tagagacttg 540

catgataatt tgtggacctt ccgccatatt tatcgcgggc aacccaaacg ccatttgcta 600

acgacagggt ggagcctctt tgtcagtggg aagaggctct ttgctggtga ctcagttttg 660

tttatcaggg atgaaaaaca gcagcttcta cttggaatta ggcgggcaaa cagacagcca 720

accaacttat catcatcggt gttgtcaagt gacagcatgc atattggtat cctagcagct 780

gctgcccatg cagctgcaaa caacagccca ttcactgtgt tttataaccc aagggcaagt 840

ccttcagaat ttgtcatccc tttagccaag tattacaagg caacttatag cagtcaagta 900

tcacttggca tgcgattccg catgatgttt gagacagaag aatcaggaac tagaaggtat 960

atgggcacaa ttactggcat cagtgatctt gatccagtga ggtggaagaa ctcacagtgg 1020

cgcaacttgc aggttggttg ggatgagtca actgctgggg agaggcgtaa ccgagtctca 1080

atttgggaga ttgaaccagt aacagcaccg tttttcatct gtcctactcc accgttcttt 1140

agatcaaagc ggccaaggct acctggaatg ccagatgatg attgttctga tctggatggc 1200

ctgtttaaga ggacaatgcc atggctcggt gatgactttg gcatgaagga tccgcaaggt 1260

cttccaggcc tgagcttagt ccaatggatg aacatgcaac agaatccttc tttggctaac 1320

tcaatgcaac cgaactacct gcattctttg tctggttccg tgctacagaa tgtaggaggt 1380

ggagcagacc tctcgcgtca gttatgtcta ccagcacctc agcttcctca gcagaatact 1440

ttgcagtttg gttcccaaag gccaacccaa caggttcagc agctcgacca gcttcagaag 1500

ataccaacta ccacattgag tccagcaggt tccattatgc agccgcagca acagttgtcg 1560

gatatcagtc agcaaccaag acagaaccta attaaccaat cagtacctac aaaccatgtt 1620

caggcccagc ttttgcaagc tcaaagtctt gtgcaatctc agaatgtcct acagcaacaa 1680

caatcatttc aaaatcagct gcaaaggaat cttcctcaga acctgccgca gcagcaacag 1740

attatgaatc aaactcagca gcaaagtttc atgcaacccc agccgagtga tccacttaac 1800

caacagctgc atttctctga taaccaatta cagatgcagc ttttgcagaa gctgcagcag 1860

cagtcacttc tagctcaaca atctttactg caacaaccgt cccagcttat gccaatccaa 1920

gatcagcaga agcacttaga tgtatcacag aacttttcta ggtcacttgc aacaagccag 1980

atgctagata tgtctcaaac cacgtccaac tccacgtctc tttcccagcc acaagttgcc 2040

cagcagcaaa tgacgataaa taatagccag tctaatcttc gttttgccca gccaaatcag 2100

catatgaagc aacagcagca gcagcagcaa ccaggaattc taccagaaat acctggacaa 2160

gttggccaga ttctaccacc aacaactaat cagctttcgg caaactgcag tagcttctta 2220

acaggagctg taggtggagg ccagtcggtg gttacggatg atattccatc atgttccacc 2280

tcaccatcta ccaacaactg tcaaaatgta gttcagccaa ttatgaatgg taggatccat 2340

cgaggcacag ctgcagcgga ggaaacaacc cagtcttctc tacccctttt gagttccagt 2400

ggattagaag ccatgtcccc taatagaaat ctagttaaag atttgcagca aaaacctgat 2460

gtgaagccct caatgaatat ctccaagagc cagaatcatg ggttttcaac tcctcaaaca 2520

taccttaata atgcagttcc ccagatggat tatttggata gttcatcttc agcaacctct 2580

gtctactttt ctcaaaatga tgtccaattg cagcagacga ctaacccgat gtcattcagt 2640

tctcaagcga tcgtatttag agatagtcaa gatggagaag ttcaaggtga ccccagacat 2700

agtgttgctt ttggagcaaa catggacaat cagttaggaa tatccatgat gcctgattct 2760

ttgatcacaa atagcttggt ggggtcaagg aaagatgtat caaataatat ctcatcaggt 2820

ggaggcatgc tttccagcta tgagaatccc aaggatgcac agcctgaact atcatcctca 2880

atggtctccc aatcatttgg agttcctgat atggccttca attcaattga ttctaccata 2940

aatgaaggca gcttcatgaa tagaggtgcc tgggccccac cacctcaaat gcctcgaatg 3000

cggacgttta ccaaggtaca caagcgtggt gctgttggaa gatcaatcga catcgcacgt 3060

tactcaggct atgaagagct taaacaagat ttagctcgta gatttggtat agagggacaa 3120

ctggaggata gacaaagaat aggatggaag cttgtatatg tggatcatga gaatgacgta 3180

ctgcttgttg gcgatgatcc ttgggaggaa ttcgtgaact gtgtccgttg catcaagatt 3240

ctttctccac aagaggtcca acaaattagc ttggatggag attttgggaa taatgtccaa 3300

aatcaagctt gtagcagttc agacgcgggg gatgtgtaa 3339

<210> 7
<211> 90
<212> RNA
<213> solanum lycopersicum

<400> 7
uucugccugg cucccuggau gccaacuaag aacaguauug ucaaaaaaau ugacacucuu 60

ccuaguuggu accagaggag acaggcagau 90